# EUROPEAN PATENT SPECIFICATION

(11) **EP 4 172 573 B1**
(45) Date of publication and mention of the grant of the patent: **05.06.2024**
(21) Application number: 20734720.4
(22) Date of filing: 24.06.2020
(51) Int. Cl.: G01D 21/00, G05D 1/00, G01N 1/00, G01N 33/00, G01N 1/02

(54) **LOCAL MEASUREMENTS USING MULTIPLE MOVING SENSORS**
LOKALE MESSUNGEN MIT MEHREREN BEWEGLICHEN SENSOREN
MESURES LOCALES À L'AIDE DE MULTIPLES CAPTEURS MOBILES

(43) Date of publication of application: 03.05.2023
(73) Proprietor: VOLVO TRUCK CORPORATION, 405 08 Göteborg (SE)
(72) Inventor: SHIRE, Joshua, 417 55 GÖTEBORG (SE)
(74) Representative: Kransell & Wennborg KB
(86) International application number: PCT/EP2020/067702
(87) International publication number: WO 2021/259469

(56) References cited:
- EP-A1- 1 113 268
- FR-A1- 2 831 665
- US-A1- 2011 163 892

## Description

### TECHNICAL FIELD

The present disclosure relates to the field of mobile measurements and in particular to the scheduling of time instants for sampling using periodically moving sensors, such as ambient sensors carried by timetabled vehicles.

### BACKGROUND

Air quality, radio conditions and other location-dependent quantities are traditionally measured using costly equipment fixedly installed at specific locations or by operating mobile laboratories. These are expensive yet coarse-grained approaches, where the measurements tend to be few and widely spaced apart. Recent attention has been given to mobile measuring techniques using vehicles already moving around an urban environment, where air quality is analyzed in real time and with low spatial granularity.

As one example, Devarakonda et al., "Real-Time Air Quality Monitoring Through Mobile Sensing in Metropolitan Areas", Proceedings of UrbComp'13 (2013) discloses air pollution sampling using public transportation vehicles and discusses sampling strategies. Further, Cruz Caminha et al., "On the Coverage of Bus-Based Mobile Sensing", Sensors, vol. 2018, issue 18, no 1976 (2018) discloses air pollution sampling using buses equipped with air pollution sensors, and discusses the balance between coverage and sensing frequency for a fleet of buses having such sensors.

The proposals by Devarakonda and Cruz Caminha both use sensors configured to measure periodically, such as twice an hour. Because the sensors are unsynchronized, both in regard to each other and to a common time base, it must be accepted that the sampling may occur anytime (in clock time) during a data collection session. This may lead to inconvenient clustering or sparseness of the samples in a given measuring location. In other words, the spatial and/or temporal coverage of the data points may be less comprehensive than their number suggests. Deficient coverage may to some extent be remedied by extending the measuring period, which however ties up the equipment unnecessarily and inflates the data set to be processed. There is a need for more intelligent coordination of mobile sensors.

FR2831665A1 discloses a system where vehicle-carried sensors sample air pollutants and report results to a central computer. Because the sensors are neither synchronized nor coordinated, the measurements to be reported must be annotated with a time and location of sampling.

US20110163892A1 discloses mobile environmental sensors which record the geographical location when sampling. A telecom base station can be used to determine where a user device has been, at any time). This reference does not disclose any active control of the sampling locations.

EP1113268A1 discloses bus-carried air quality sensors. The position of a sensor is determined just after it has sampled the air quality. The sampling positions are not controllable, however, nor are the sensors aware of the geographical area in which the air quality monitoring shall be conducted.

### SUMMARY

One objective is to make available methods and devices for controlling sampling at a plurality of mobile ambient sensors, which move periodically along predefined routes. A further objective is to address inherent limitations of the sensors, such as refractory periods.

These and other objectives are achieved by the invention defined by the independent claims. The dependent claims relate to embodiments of the invention.

One aspect of the invention relates to a method of controlling sampling at a plurality of mobile ambient sensors. The method comprises providing a plurality of ambient sensors, each of which moves periodically along a predefined route. Different sensors may travel along different routes. The method further comprises receiving one or more desired measuring locations. The measuring locations may be input by a user, transferred from an automated or semi-automated planning tool or obtained in another suitable way. The method then assigns a first sampling location to a first one of the sensors. The first sampling location shall be at or near the desired measuring location, such as on the route along which the first sensor moves.

This method allows precise control of the actual sampling locations of the different ambient sensors and allows coordination of multiple sensors. The spatial clustering effect seen in the state-of-the-art measuring techniques can be easily avoided, because the first sensor is set to sample at the first sampling location, any second sensor is set to sample at a second sampling location, which can be configured independently of the first sampling location, and so forth. The method can also be used to eliminate the occurrence of unmeasured spatial gaps at locations of particular interest, as long as such locations are situated on one of the routes.

A further benefit is that time-wise clustering of samples - consecutive measurements having too small spacing - can be controlled or even avoided with the present method. More precisely, if the sensors are moving steadily and no two different sensors are assigned identical sampling locations, the next measurement at a given sampling location will not occur until the sensor has completed its route and returns to the sampling location. Consecutive measurements by this sensor will therefore be inherently spaced in time.

In one embodiment, a second one of the mobile ambient sensors is assigned a sampling location, termed second sampling location, which like the first sampling location is at or near the same desired measuring location. This allows precise control of both sensors and more complete coverage of the measuring location. This embodiment is not limited to the use of two ambient sensors, but may extend to three, four or more sensors.

In one embodiment, a sampling location is assigned to a sensor together with a sampling periodicity. The periodicity governs the behavior of the sensor if it remains at the sampling location for a non-zero duration. The sensor may so remain if its movement is interrupted (e.g., if the vehicle carrying the sensor is loading or unloading, or halting at traffic signals) or if the sampling location has a spatial extent (e.g., area, road segment, point with a proximity zone). One use of the option to assign a sampling periodicity is to avoid time clustering of measurements.

Another benefit of assigning a sampling periodicity is the ability to account for refractory periods. A refractory period of a sensor is an amount of time which must elapse between two consecutive sampling instances of the sensor. A sensor may be associated with a non-zero refractory period as a result of its measuring principle (e.g., maximum readout frequency of photoelectric elements), downstream resources (e.g., throughput of signal processing circuitry) or a necessary adaptation to a new location to which it is moved (e.g., by allowing a measuring chamber time to purge and refill with ambient air). A sensor with a non-zero refractory period is normally unable of continuous operation at its nominal accuracy. According to this embodiment, the sampling periodicity is assigned in view of the refractory period. This may signify that the sampling periodicity must not be shorter than the refractory period. In one implementation, the sampling periodicity is set equal or approximately equal to the refractory period; this may maximize the dataset collected by that sensor in given time.

In one embodiment, where each of the sensors is associated with a refractory period in the sense described above, one sensor is assigned at least two sampling locations, which may correspond to respective desired measuring locations. The spacing of the sampling locations is selected in view of the refractory period and the route of the sensor. A "spacing" in this sense may be a time separation according to a timetable for the route of the sensor or a distance, a spatial separation. The two sampling locations must not be closer (as measured along the route) than the product of the sensor's refractory period and the speed at which the sensor moves along the route. It is advantageous for the method according to this aspect of the invention to control the plurality of mobile ambient sensors in a coordinated manner. Such coordination may involve that the 'global' task of assigning all sensors' sampling locations optimally in relation to the received desired measuring location is prioritized over the 'local' task of maximizing the use of each sensor. The 'local' task may be solved by operating each sensor with a periodicity close to its refractory period, but this behavior may have to yield to the prioritized goal of finding a globally optimal sampling location assignment.

In one embodiment, a processor executing the method receives data representing a route and/or a refractory period of each of the sensors and uses it for performing the assigning step. Such data may be received from the sensor itself, if communication-enabled, or from an entity associated with the sensor, such as a communication-enabled vehicle or vessel on which the sensor is mounted. A further option is for an administrator to collect the corresponding data based on known hardware characteristics, traffic schedules or the like, and provide it to said executing processor. An advantage with the first option, may be that the person who defines the desired measuring locations (e.g., an environment analyst, a radio spectrum regulator) may not have convenient access to up-to-date public transport timetables or sensor hardware.

In a further development of this embodiment, a sensor or entity associated with the sensor creates the data representing the sensor's route by recording historic geolocations and discerning periodic patterns therein. This avoids any need to convert public transport timetables into routes represented in a computer-readable format, and the need to update the routes when timetables change.

It is understood that the method according to the first aspect may be performed at a controller, which has the authority to control the mobile ambient sensors directly or indirectly. As an example of indirect control, the controller may output an instruction needing to be forwarded to the sensors by a third party, such as an administrator. As a further example, the controller may be authorized to upload such instruction to the respective sensors, which however are configured to not execute the instruction unless an administrator transmits an authorization command to the sensors. The controller may be associated with one of the sensors (e.g., installed in a vehicle that carries one of the sensors) or may be centralized in the sense of not being associated with any sensor (e.g., installed a stationary fashion or in a vehicle not carrying any sensor). The controller may be entrusted with the further task of collecting data from the sensors and processing the data.

For these reasons, the act of "providing a plurality of ambient sensors" may have different meanings depending on properties of the controller executing the method. It is possible to execute the method as soon as information about the routes of the sensors is available and the at least one desired measuring location is known; the assigned sampling locations may be output in the form of an instruction to be forwarded to the sensors. In implementations where another party controls the sensors, "providing" may include receiving such information regarding the sensors and possibly being granted access rights for communicating with or controlling the sensors. In other implementations, "providing" may include commissioning, deploying and/or operating the sensors.

A controller suitable for the above purposes may comprise an interface configured to receive one or more desired measuring locations. The controller may further comprise processing circuitry configured to assign to a first one of the sensors a first sampling location, which is at or near the desired measuring location.

The invention further relates to a computer program containing instructions for causing a computer, or the controller in particular, to carry out the above method. The computer program may be stored or distributed on a data carrier. As used herein, a "data carrier" may be a transitory data carrier, such as modulated electromagnetic or optical waves, or a non-transitory data carrier. Non-transitory data carriers include volatile and non-volatile memories, such as permanent and non-permanent storages of magnetic, optical or solid-state type. Still within the scope of "data carrier", such memories may be fixedly mounted or portable.

As used herein, a "location" maybe geographical point or area, a road segment or a region of three-dimensional space. An "ambient sensor" is one configured to measure a physicochemical, optical, electromagnetic, biological or another location-dependent quantity. If such sensor is carried by a road or railroad vehicle, sea or air vessel or the like, then in normal circumstances the measured quantity is unrelated to the operation of the vehicle or vessel itself or only marginally perturbed by it (e.g., air quality sensor occasionally sensing vehicle's exhaust gases, cellular coverage sensor capturing reflections when metallic body parts align with base station antennas). Two or more ambient sensors carried by the same vehicle or vessel, which are therefore associated with identical routes, can nevertheless be treated independently in the present method, in particular as regards the assignment of sampling locations. For example, the sampling locations and sampling periodicity assigned to a one sensor need not coincide with those of a further sensor.

Generally, all terms used in the claims are to be interpreted according to their ordinary meaning in the technical field, unless explicitly defined otherwise herein. All references to "a/an/the element, apparatus, component, means, step, etc." are to be interpreted openly as referring to at least one instance of the element, apparatus, component, means, step, etc., unless explicitly stated otherwise. The steps of any method disclosed herein do not have to be performed in the exact order disclosed, unless explicitly stated.

### BRIEF DESCRIPTION OF THE DRAWINGS

Aspects and embodiments are now described, by way of example, with reference to the accompanying drawings, on which:
figure 1 is a flowchart of a method for controlling sampling at a plurality of mobile ambient sensors according to an embodiment of the invention;
figure 2 is a schematic drawing of sensors moving along predefined routes which overlap with desired measuring locations;
figure 3 shows a vehicle carrying two ambient sensors and is in wireless communication with a stationary controller; and
figure 4 shows example vehicles suitable for carrying mobile ambient sensors.

### DETAILED DESCRIPTION

The aspects of the present disclosure will now be described more fully hereinafter with reference to the accompanying drawings, on which certain embodiments of the invention are shown. These aspects may, however, be embodied in many different forms and the embodiments should not be construed as limiting; rather, they are provided by way of example so that this disclosure will be thorough and complete, and to fully convey the scope of all aspects of invention to those skilled in the art. Like numbers refer to like elements throughout the description.

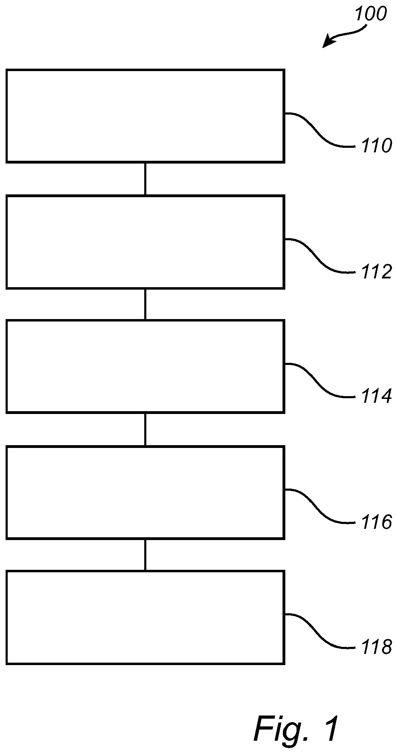

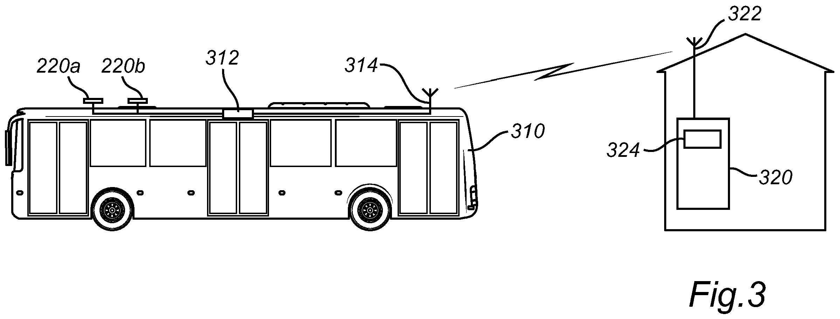

Figure 1 is a flowchart of a method 100 for controlling sampling at a plurality of mobile ambient sensors according to an embodiment of the invention. In what follows, the method 100 is will be described when implemented by a controller 320 of the general type shown in the right portion of figure 3.

The controller 320 may be a programmable general-purpose computer equipped with or connected to a communication interface towards the ambient sensors to be controlled. The controller 320 in this embodiment does not move with any of the ambient sensors during the execution of the method 100. In figure 3, it is shown located in a building. It is well known that a general-purpose computer includes memory, processing circuitry 324 and a data interface for inputting and outputting data. While the communication interface is drawn simplistically in figure 3, as a rooftop antenna 322 directly communicating with the ambient sensors, it is understood that the communication interface may be a connection to a cellular network, where the actual radio link to a sensor is established from a radio base station managed by a network operator. As mentioned above, the communication interface towards the ambient sensors is not essential to allow execution of the method 100; the programming of the sensors in accordance with the method's 100 output data may be entrusted to another party.

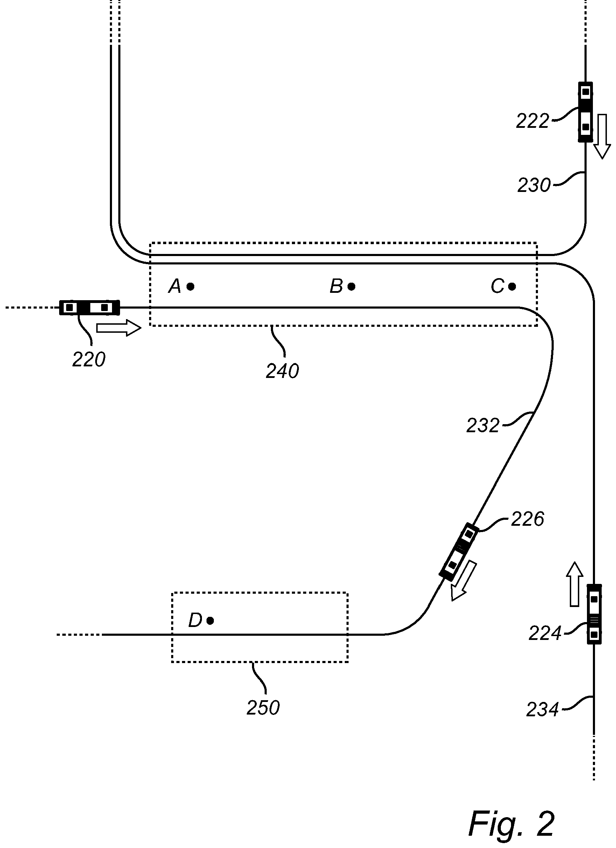

Referring to a first step 110 of the method 100, a plurality of ambient sensors 220, 222, 224, 226 are illustrated in figure 2. Each sensor moves periodically along a predefined route. The periodical movement of the sensors may include repeated linear movement from an initial point to a final point, reciprocating movement (back and forth) between two endpoints, or cyclic movement along a closed curve. Here, sensor 222 moves along a first route 230, sensors 220 and 226 move along a second route 232, and sensor 224 moves along a third route 234. For clarity of the drawing, only portions of the first, second and third routes 230, 232, 234 are shown in figure 2. It is seen that some segments of the routes 230, 232, 234 coincide or run parallel. Because measurements in locations at or near these segments can be effectuated by a freely selectable one of the corresponding sensors, the method 100 has room to optimize the precise sampling locations and thereby the relative timing.

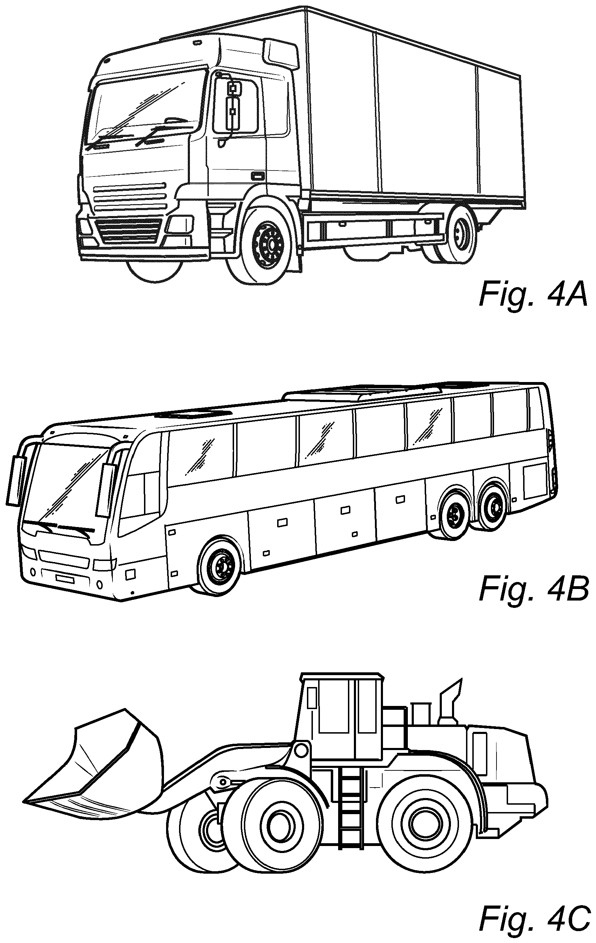

The sensors 220, 222, 224, 226 are mounted on geolocation-enabled vehicles, such as the truck, bus and construction equipment shown in figure 4. The vehicles may be conventional or autonomous (driverless). Generally speaking, "geolocation-enabled vehicles" includes vehicles and vessels with a Global Navigation Satellite Systems (GNSS) receiver, a cellular transceiver positionable in relation to base stations, a wireless local area network receiver positionable on the basis of a known access point location, an inertial measurement unit or other positioning equipment. Sensors which are associated with a positioning functionality are controllable on the basis of location. For example, each sensor may be configured to sample when it receives a signal from the vehicle, on which it is mounted, that it is currently at or near a sampling location assigned to the sensor. Alternatively, the sensor may receive updated geolocation data from the vehicle (or poll the vehicle for such data) and evaluate it against the sampling location(s) assigned to it. As a further alternative, the sensor may be geolocation-enabled in itself; at a possibly higher per-sensor cost, this avoids the reliance on the vehicle's positioning resources and the need to interface with these.

In the present example, the bus 310 shown in the left portion of figure 3 acts as the geolocation-enabled vehicle. The bus carries two sensors 220a, 220b which may for example be urban air quality sensors designed to measure nitrogen oxide pollutants (NOₓ) and particulate matter (PM), respectively. The sensors are connected to an internal controller 312 of the bus 310. The internal controller 312 serves as an interface towards resources shared by the bus 310, such as electric power, compressed air, a time signal, positioning services, data connectivity and the like. As suggested by an external antenna 314 in figure 3, the internal controller 312 can communicate wirelessly with the controller 320.

The bus 310 is assumed to be a public transport vehicle serving a route defined in a public timetable, so that the sensors 220a, 220b will move periodically along this route. It is noted that many vehicle types operate according to non-public timetables or have periodic movement patterns for other reasons, which can therefore replace the bus 310 in this example. This includes refuse collection trucks, postal vehicles, street sweepers, mining and construction vehicles.

From the point of view of the sampling control method 100, the step 110 of providing the ambient sensors 220, 222, 224, 226 includes obtaining up-to-date information indication the numbers and identities of the active sensors and their measuring capabilities.

In a second step 112, the controller 320 receives the desired measuring locations 240, 250. One may therefore consider that a purpose of the method 100 is to enable the efficient the collection of air quality measurements here. As shown in figure 2, the two desired measuring locations 240, 250 in this example have a two-dimensional geometry.

In a next step 114, the controller 320 receives data from each sensor 220, 222, 224, 226 representing a route and any refractory period of the sensor. Based on this data, the controller 320 is able to ascertain which ones of the routes 230, 232, 234 can be used to cover each of the desired measuring locations 240, 250. In the situation illustrated in figure 2, sensors traveling on all three routes 230, 232, 234 can measure in the first desired measuring location 240, while only the second route 232 reaches the second desired measuring location 250.

In steps 116 and 118, to cover the first measuring location 240, the controller 320 assigns sampling location A to the second sensor 222; sampling location B to the first and third sensors 220, 224; and sampling location C to the second and third sensors 222, 224. (It is noted that sampling location B is assigned to either sensor 220a or sensor 220b. Certainly these are carried by the same vehicle 310 but are treated as separate sensors for the purposes of this method 100; this is all the more justified as the sensors 220a, 200b are configured to measure different quantities.) To cover the second measuring location 250, a sampling location D is assigned to the fourth sensor 226. One or more of the sampling locations may optionally be assigned in conjunction with a sampling periodicity.

The resulting sampling times follow from the movements of the sensors. In the present example, figure 2 shows the positions and directions of the sensors at an instant between time *t* = *n* - 1 (arbitrary units) and *t* = *n.* The approximate sampling times for the respective sensors are given in Table 1.

| Table 1 | | | | | |
|---|---|---|---|---|---|
| | n-1 | n | n+1 | n+2 | n+3 |
| 220 | | | B | | |
| 222 | A | C | | B | |
| 224 | | B | | A | C |
| 226 | | | | D | |

In other embodiments, a corresponding overview of the sampling times may be obtained from timetables or other service plans, possibly with the aid of simulations modeling the effects of non-deterministic traffic flows, weather conditions, boarding and alighting passengers, and other factors causing timetable deviations.

It is derivable from the present Table 1, first, that the time-wise coverage of the first measuring location 240 is adequate, for at least one sample will be taken at each of the shown time instants. The coverage of the second measuring location 250 may be considered optimal in the circumstances, knowing that only the fourth sensor 226 is available but that it is utilized (once, or according to an assigned sampling periodicity) whenever it passes.

Second, Table 1 allows to establish whether each sensor's refractory period has been observed. The separation of two sampling times by the first and fourth sensors 220, 226 is at least 5 time units. The corresponding number for the second and third sensors 222, 224 is 1 time unit. If this is incompatible with the refractory periods reported in step 114, then the sampling locations A, B, C (and possibly D) are discarded and steps 116-118 repeated until a satisfactory assignment is found.

Steps 116-118 may be repeated by initiating another execution of a random procedure, an optimization solver run with slightly different initial values, or with another change allowing a different number of sampling locations or differently situated sampling locations to be objectively expected. Another approach to the removal of too dense sampling is to apply modifications at the problematic time instants directly. For the second sensor 222, such a time instant may be *t* = *n,* where sampling at location C will occur only one time unit after the sampling at location B; giving up sampling location C may be a viable option, as the first measuring location 240 is anyway covered by the third sensor 224 at sampling location B at *t* = *n.* Similarly, it may be justified to un-assign sampling location A from the third sensor 224 at *t* = *n* + 2, which may jeopardize this sensor's ability to sample at location C one time unit later.

As explained in an earlier section of this disclosure, clustering of measurements is to be avoided. Table 2 allows to examine to what extent this goal is achieved by an assignment of sampling locations.

| Table 2 | | | | |
|---|---|---|---|---|
| | A | B | C | D |
| 220 | | n+1 | | |
| 222 | n-1 | n+2 | n | |
| 224 | n+2 | n | n+3 | |
| 226 | | | | n+2 |

It is clear from Table 1 that there are no simultaneous measurements at any of the sampling locations. The sensitivity to clustering is related to the time unit used. Executing the method 100 with a longer time unit will correspond to a lower time resolution (higher granularity), which tends to identify a greater number of simultaneous measurements. The method 100 will then reject and recompute candidate sampling location assignments relatively more often, so that the clustering tendency is suppressed more vigorously.

The steps 116-118 may optionally be followed by a programming step and/or a data collection step. In the programming step, the sensors 220, 222, 224, 226 are controlled in accordance with the sampling locations A, B, C, D. In the data collection step, the controller 320 or another data recipient receives the measurement results from the sensors.

In a variation of the described embodiment, the method 100 may be executed by the internal controller 312. The beneficiary of the collected data may then be the vehicle 310 or a person using or traveling with the vehicle 310. The internal controller 312 may be elected as lead controller by the execution of a leader election algorithm by the simultaneously present sensors associated with analogous controller equipment; the leader election algorithm may be random or pseudo-random. When the method 100 is executed from an internal controller 312 of a vehicle, communication with other sensors (possibly, through the intermediary of the vehicles on which they are mounted) may proceed over a cellular network or short-range wireless. Multi-hop or 'meshed' communication, by which a nearby vehicle relays a message towards a more distant vehicle, is a way to alleviate the coverage limitations of short-range wireless, so as to increase the workable size of a measuring zone.

While clearly not essential to the method 100, some heuristics and automated approaches for assigning the sampling locations (and any sampling periodicities) on the basis of desired measuring locations and refractory periods will now be discussed. One possible approach is to perform a random or pseudo-random assignment of sampling locations, which is evaluated against predefined criteria and repeated if needed. The criteria may include:
- completeness of time coverage,
- avoidance of clustering,
- observance of refractory periods,
- completeness of spatial coverage of the desired measuring locations,
- resilience (e.g., the number of independent sensors that are engaged to cover each measuring location).

As explained above, the three first criteria may be evaluated by forming Tables 1 and 2 for the case under consideration.

Another possible approach is to formulate an optimization problem which is solved numerically, with the sampling locations as output. The above criteria may either be included in an objective function or in boundary conditions. For example, the objective function may be dependent on the sampling locations and may include terms penalizing clustering and rewarding complete coverage. The refractory periods, which are arguably of a more coercive character, may be included as boundary conditions. The objective function of such an optimization problem may further include a term rewarding the total number of samples collected, so as to reflect a desire to optimize the use of a given number of sensors. The choice of the numerical solver is not essential to the present invention; it will be within the skilled person's abilities to select a suitable one of the solvers described in the literature.

Machine learning methods constitute a still further option for automating the assignment steps 116-118.

The aspects of the present disclosure have mainly been described above with reference to a few embodiments. However, as is readily appreciated by a person skilled in the art, other embodiments than the ones disclosed above are equally possible within the scope of the invention, as defined by the appended patent claims.

## Claims

1. A method (100) of controlling sampling at a plurality of mobile ambient sensors, comprising:
providing (110) a plurality of ambient sensors (220, 222, 224, 226), wherein each sensor moves periodically along a predefined route (230, 232, 234) and is controllable on the basis of location by being associated with a positioning functionality;
receiving (112) one or more desired measuring locations (240, 250), wherein said predefined routes overlap with the desired measuring locations; and
assigning (116) to a first one of the sensors a first sampling location (A, B, C, D), which is at or near the desired measuring location, wherein the assigned sampling locations are output in the form of an instruction to be forwarded to the sensors.

2. The method of claim 1, wherein said assigning further comprises:
assigning (118) to a second one of the sensors a second sampling location (A, B, C, D), which is at or near the same desired measuring location.

3. The method of any of the preceding claims, wherein said assigning (116, 118) includes setting a sampling periodicity to apply at the first or second sampling location.

4. The method of claim 3, wherein:
each of the sensors is associated with a predefined refractory period, which must elapse between two consecutive sampling instances; and
the sampling periodicity is set in view of the refractory period.

5. The method of any of the preceding claims, wherein:
each of the sensors is associated with a predefined refractory period, which must elapse between two consecutive sampling instances; and
at least two sampling locations are assigned to one sensor, wherein the separation of the sampling locations is selected in view of the refractory period and the route of the sensor.

6. The method of claim 5, wherein the at least two sampling locations correspond to respective desired measuring locations.

7. The method of any of the preceding claims, further comprising:
receiving (114), from each sensor or an entity associated with the sensor, data representing a route and/or a refractory period of the sensor.

8. The method of any of the preceding claims, which is performed by a controller (320) not associated with any sensor, wherein data representing the assigned sampling locations is distributed to each concerned sensor by wireless communication.

9. The method of any of the preceding claims, wherein the sensors are mounted on geolocation-enabled vehicles (310), in particular timetabled geolocation-enabled vehicles.

10. The method of claim 9, wherein one vehicle carries multiple sensors, to which sampling locations are assigned independently.

11. A controller (312, 320) operable to control a plurality of mobile ambient sensors (220, 222, 224, 226), wherein each sensor moves periodically along a predefined route (230, 232, 234) and is controllable on the basis of location by being associated with a positioning functionality, the controller comprising:
an interface configured to receive one or more desired measuring locations (240, 250), wherein said predefined routes overlap with the desired measuring locations; and
processing circuitry (324) configured to assign to a first one of the sensors a first sampling location (A, B, C, D), which is at or near the desired measuring location, wherein the assigned sampling locations are in the form of an instruction to the sensors.

12. A computer program comprising instructions to cause the controller of claim 11 to perform the method of any of claims 1 to 10.

13. A data carrier with the computer program of claim 12.

## Patentansprüche

1. Verfahren (100) zur Steuerung der Abtastung von mehreren mobilen Umgebungssensoren, umfassend:
Bereitstellen (110) mehrerer Umgebungssensoren (220, 222, 224, 226), wobei sich jeder Sensor periodisch entlang einer vordefinierten Route (230, 232, 234) bewegt und auf der Grundlage des Standorts steuerbar ist, indem er mit einer Positionierungsfunktionalität verbunden ist;
Empfangen (112) eines oder mehrerer gewünschter Messorte (240, 250), wobei sich die vordefinierten Routen mit den gewünschten Messorten überschneiden; und Zuordnen (116) eines ersten Abtastortes (A, B, C, D), der sich am oder in der Nähe des gewünschten Messortes befindet, an einen ersten der Sensoren, wobei die zugeordneten Abtastorte in Form einer an die Sensoren zu übermittelnden Anweisung ausgegeben werden.

2. Verfahren nach Anspruch 1, wobei das Verfahren ferner umfasst:
Zuordnen (118) eines zweiten Sensors zu einem zweiten Abtastort (A, B, C, D), der sich am oder in der Nähe des gleichen gewünschten Messortes befindet.

3. Verfahren nach einem der vorhergehenden Ansprüche, wobei das Zuordnen (116, 118) das Einstellen einer Abtastperiodizität umfasst, die an dem ersten oder zweiten Abtastort anzuwenden ist.

4. Verfahren nach Anspruch 3, wobei:
jeder der Sensoren mit einer vordefinierten Refraktärzeit verbunden ist, die zwischen zwei aufeinanderfolgenden Abtastzeitpunkten vergehen muss, und
die Abtastperiodizität unter Berücksichtigung der Refraktärzeit festgelegt wird.

5. Verfahren nach einem der vorhergehenden Ansprüche, wobei:
jeder der Sensoren mit einer vordefinierten Refraktärzeit verbunden ist, die zwischen zwei aufeinanderfolgenden Abtastzeitpunkten vergehen muss, und
einem Sensor mindestens zwei Abtastorte zugeordnet sind, wobei der Abstand der Abtastorte im Hinblick auf die Refraktärzeit und der Route des Sensors gewählt wird.

6. Verfahren nach Anspruch 5, wobei die mindestens zwei Abtastorte den jeweiligen gewünschten Messorten entsprechen.

7. Verfahren nach einem der vorhergehenden Ansprüche, ferner umfassend:
Empfangen (114) von Daten, die eine Route und/oder eine Refraktärzeit des Sensors darstellen, von jedem Sensor oder einer mit dem Sensor verbundenen Einheit.

8. Verfahren nach einem der vorhergehenden Ansprüche, das von einem Steuergerät (320) durchgeführt wird, das keinem Sensor zugeordnet ist, wobei Daten, die die zugeordneten Abtastorte repräsentieren, durch drahtlose Kommunikation an jeden betroffenen Sensor verteilt werden.

9. Verfahren nach einem der vorhergehenden Ansprüche, wobei die Sensoren an geolokalisierungsfähigen Fahrzeugen (310), insbesondere an zeitgesteuerten geolokalisierungsfähigen Fahrzeugen, angebracht sind.

10. Verfahren nach Anspruch 9, wobei ein Fahrzeug mit mehreren Sensoren ausgestattet ist, denen unabhängig voneinander Abtastorte zugeordnet sind.

11. Steuergerät (312, 320) betreibbar, um mehrere mobile Umgebungssensoren (220, 222, 224, 226) zu steuern, wobei sich jeder Sensor periodisch entlang einer vordefinierten Route (230, 232, 234) bewegt und auf der Grundlage des Standorts steuerbar ist, indem er mit einer Positionierungsfunktionalität verbunden ist, umfassend:
eine Schnittstelle, die konfiguriert ist, um einen oder mehrere gewünschte Messorte (240, 250) zu empfangen, wobei sich die vordefinierten Routen mit den gewünschten Messorten überschneiden; und
Verarbeitungsschaltung (324), die konfiguriert ist, um einem ersten der Sensoren einen ersten Abtastort (A, B, C, D) zuzuordnen, der sich an oder in der Nähe des gewünschten Messortes befindet, wobei die zugeordneten Abtastorte in Form einer Anweisung an die Sensoren vorliegen.

12. Computerprogramm mit Anweisungen, um das Steuergerät nach Anspruch 11 zu veranlassen, das Verfahren nach einem der Ansprüche 1 bis 10 durchzuführen.

13. Datenträger mit dem Computerprogramm nach Anspruch 12.

## Revendications

1. Procédé (100) de commande d'échantillonnage sur une pluralité de capteurs ambiants mobiles, comprenant :
la prévision (110) d'une pluralité de capteurs ambiants (220,222,224,226), chaque capteur se déplaçant périodiquement le long d'un itinéraire prédéfini (230,232,234) et étant contrôlable sur la base de son emplacement en étant associé à une fonctionnalité de positionnement ;
la réception (112) d'un ou plusieurs emplacements de mesure souhaités (240,250), chaque itinéraire prédéfini se chevauchant avec des emplacements de mesure souhaités ; et
l'affectation (116) à un premier des capteurs d'un premier emplacement d'échantillonnage (A, B, C, D) qui est à ou proche de l'emplacement de mesure souhaité, les emplacements échantillonnage affectés étant émis sous la forme d'une instruction à envoyer aux capteurs.

2. Procédé selon la revendication 1, dans lequel ladite affectation comprend en outre :
l'affectation (118) à un second des capteurs d'un second emplacement d'échantillonnage (A, B, C, D) qui est à ou proche du même emplacement de mesure souhaité.

3. Procédé selon l'une quelconque des revendications précédentes, dans lequel l'affectation (116,118) inclut la définition d'une périodicité d'échantillonnage à appliquer au premier ou second emplacement d'échantillonnage.

4. Procédé selon la revendication 3, dans lequel :
chacun des capteurs est associé à une période réfractaire prédéfinie qui doit s'écouler entre deux instances d'échantillonnage consécutives ; et
la périodicité d'échantillonnage est établie en vue de la période réfractaire.

5. Procédé selon l'une quelconque des revendications précédentes, dans lequel
chacun des capteurs est associé à une période réfractaire prédéfinie qui doit s'écouler entre deux instances d'échantillonnage consécutives ; et
au moins deux emplacements d'échantillonnage sont affectés à un capteur, la séparation des emplacements d'échantillonnage étant sélectionnée en vue de la période réfractaire et de l'itinéraire du capteur.

6. Procédé selon la revendication 5, dans lequel les au moins deux emplacements d'échantillonnage correspondent à des emplacements de mesure souhaités.

7. Procédé selon l'une quelconque des revendications précédentes, comprenant en outre :
la réception (114), de la part de chaque capteur ou d'une entité associée au capteur, de données représentant un itinéraire et/ou une période réfractaire du capteur.

8. Procédé selon l'une quelconque des revendications précédentes, qui est réalisé par une commande (320) non associée à un capteur, les données représentant les emplacements des échantillonnages affectés étant réparties vers chaque capteur concerné par communication sans fil.

9. Procédé selon l'une quelconque des revendications précédentes, dans lequel les capteurs sont montés sur des véhicules à géolocalisation activée (310), en particulier des véhicules à géolocalisation activée à horaires programmés.

10. Procédé selon la revendication 9, dans lequel un véhicule transporte de multiples capteurs auxquels des emplacements échantillonnage sont affectés indépendamment.

11. Commande (312,320) actionnable pour commander une pluralité de capteurs ambiants mobiles (220,222,224,226), chaque capteur se déplaçant périodiquement le long d'un itinéraire prédéfini (230,232,234) et étant contrôlable sur la base de son emplacement en étant associé à une fonctionnalité de positionnement, la commande comprenant :
une interface configurée pour recevoir un ou plusieurs emplacements de mesure souhaités (240,250), lesdits itinéraires prédéfinis se chevauchant avec les emplacements de mesure souhaités ; et
des circuits de traitement (324) configurés pour affecter à un premier des capteurs un premier emplacement d'échantillonnage (A, B, C, D) qui est à ou proche de l'emplacement de mesure souhaité, les emplacements d'échantillonnage affectés étant émis sous la forme d'une instruction à envoyer aux capteurs.

12. Programme informatique comprenant des instructions pour amener la commande selon la revendication 11 à réaliser le procédé selon l'une quelconque des revendications 1 à 10.

13. Support de données comportant le programme informatique selon la revendication 12.
